# EUROPÄISCHE PATENTSCHRIFT

(11) **EP 2 181 729 B1**
(45) Veröffentlichungstag und Bekanntmachung des Hinweises auf die Patenterteilung: **21.03.2012**
(21) Anmeldenummer: 09172870.9
(22) Anmeldetag: 13.10.2009
(51) Int. Cl.: A61N 1/372

(54) **Modulares Universalprogrammiergerät**
Modular universal programmer
Programmateur universel modulaire

(30) Priorität: 04.11.2008 DE 102008043451
(43) Veröffentlichungstag der Anmeldung: 05.05.2010
(73) Patentinhaber: Biotronik CRM Patent AG, 6341 Baar (CH)
(72) Erfinder: Dörr, Thomas, 12437, Berlin (DE); Hennig, Carsten, 13591, Berlin (DE); Elsner, Joachim, 14059, Berlin (DE); Hensen, Kai, 52428, Jülich (DE); Dodt, Torsten, 10439, Berlin (DE)
(74) Vertreter: Lindner-Vogt, Karin L.

(56) Entgegenhaltungen:
- WO-A2-2008/042610
- US-A- 5 800 473
- US-B2- 7 239 918
- Medtronic, Inc.: "Radio Frequency Considerations for the Use of Wireless Telemetry" 1. Mai 2006 (2006-05-01), XP002565287 Gefunden im Internet: URL:http://www.medtronic.ch/physician/hf/R adioFreqWirelessTelemetryWhitePaper.pdf> [gefunden am 2010-01-23]
- STIRBYS PETRAS: "A Challenge: Development of a Universal Programmer" PACE, Bd. 16, Nr. 4, 1. April 1993 (1993-04-01), Seiten 693-694, XP002565288 DOI: 10.1111/j.1540-8159.1993.tb01644.x

## Beschreibung

Die Erfindung betrifft ein universales Programmiergerät für patientenindividuelle Medizingeräte wie Implantate.

In WO 2008/042610 A2 ist ein Programmiergerät beschrieben, welches aus mit einem Computer gekoppelten herstellerspezifischen Telemetriemodul besteht. In US 7,209,790B2 ist ein Multi-Mode Universalprogrammiergerät beschrieben, das allerdings nicht im gewünschten Maße herstellerübergreifend kompatibel ist.

Die der Erfindung zugrunde liegende Aufgabe besteht darin, ein Gerät zu schaffen, mit dem elektronische Implantate verschiedener Hersteller zu identifizieren, in Notfallsituationen zu steuern oder vollständig nachzusorgen sind.

Erfindungsgemäß wird diese Aufgabe durch ein Programmiergerät der eingangs genannten Art gelöst, das einen RF-Transceiver (Sender/Empfänger), eine Steuereinrichtung und ein Mensch-Maschine-Interface oder einen Anschluss für das Mensch-Maschine-Interface aufweist. Der RF-Transceiver ist zum Empfang und zur Übertragung von Daten im MICS-Band ausgebildet. Die Steuereinrichtung ist mit dem Transceiver verbunden und weist derart vorkonfigurierte Softwareschnittstellen auf, dass das Programmiergerät mit Steuersoftwaremodulen erweiterbar ist, wobei die vorkonfigurierten Softwareschnittstellen eine einheitliche Schnittstelle zur Ansteuerung des Transceivers definieren, auf die Steuersoftwaremodule zugreifen können. Die Mensch-Maschine-Schnittstelle, beispielsweise eine Tastatur und/oder ein Display oder der Anschluss für eine Mensch-Maschine-Schnittstelle, ist mit der Steuereinrichtung verbunden.

Die Erfindung wird durch Anspruch 1 definiert.

Das MICS-Frequenzband ist für Medical Implant Communication Services, also die Datenkommunikation mit medizinischen Implantaten, vorgesehen und belegt Frequenzen zwischen 402 und 405 MHz.

Der RF-Transceiver ist derart ausgebildet, dass er für Modulations- und Protokollverfahren im MICS-Band geeignet ist und herstellerspezifische MICS-Protokolle unterstützen kann. Der RF-Transceiver ist somit ein Softwareprogrammierbares MICS Radio.

Die Steuereinrichtung weist einheitliche Hardware-Schnittstellen für hersteller-spezifische Hardware-Zusatzmodule auf, wobei die Hardware-Schnittstellen über die Steuereinrichtung mit dem RF-Transceiver verbunden sind.

Die vorkonfigurierten Softwareschnittstellen sind vorzugsweise derart konfiguriert, dass die elektronischen Implantate verschiedener Hersteller durch Hinzufügen von herstellerspezifischen Softwarekomponenten mindestens identifiziert werden können.

Das Programmiergerät weist eine oder mehrere Antennen zur MICSKommunikation auf, die mit dem RF-Transceiver verbunden sind.

Die Hardware-Zusatzmodule enthalten eine Treibersoftware zur Ansteuerung der Antennen. Die Treibersoftware ermöglicht die Realisierung eines jeweiligen RF-Protokolls.

Die Hardware-Zusatzmodule beinhalten vorzugsweise bereits eine jeweilige herstellerspezifische Software, die Komponenten für ein grafisches Benutzerinterface (GUI) zur Wiedergabe über die Mensch-Maschine-Schnittstelle einschließt.

Vorzugsweise weist die Mensch-Maschine-Schnittstelle mindestens ein Display zur Identifikation eines elektronischen Implantates auf, das beispielsweise zur Anzeige einer Seriennummer eines Implantats oder eines Patientennamens ausgebildet ist.

Ebenso ist bevorzugt, dass die Mensch-Maschine-Schnittstelle eine oder mehrere Tasten aufweist, die zur Auslösung implantatspezifischer Notfallfunktionen mit der Steuereinrichtung verbunden sind. Mit derartigen Tasten lassen sich z.B. ein Notschock bei implantierbaren Kardiovertern/Defibrillatoren (ICDs) oder ein Schutzprogramm bei implantierbaren Pulsgeneratoren (IPGs) wie Herzschrittmachern oder ein "AUS" bei Medikamentenpumpen auslösen, so dass das Programmiergerät auch ohne zusätzliche Netzwerkterminals für den Notfalleinsatz genutzt werden kann.

Vorzugsweise weist das Programmiergerät zusätzliche Frequenzgeneratoren für eine Datenkommunikation in anderen Frequenzbereichen als dem MICS-Frequenzbereich auf. Dies erlaubt z.B. die Aktivierung der MICS-Kommunikation durch vorangehende Signalisierung in anderen Frequenzbändern als dem MICS-Frequenzband. Diese anderen Frequenzbänder können im Bereich von 32 kHz bis max. 2,5 GHz liegen. Dies erlaubt es, die Datenkommunikation im MICS-Frequenzbereich nur dann zu aktivieren, wenn diese Datenkommunikation benötigt wird.

Vorzugsweise weist das Programmiergerät diesem fest zugeordnete Softwareframeworks und definierte Schnittstellen zu Steuersoftwaremodulen auf, so dass herstellerspezifische Steuersoftwaremodule in dem Programmiergerät mittels eines Softwareframeworks, wie z.B. "COM" oder ".net", eingebettet werden können.

Vorzugsweise besitzt das Programmiergerät eine oder mehrere Schnittstellen zu einem oder mehreren Terminals zur Steuerung der Funktionen durch einen Anwender, wie z.B. einen Arzt. Hierzu ist das Programmiergerät vorzugsweise so ausgebildet, dass die Terminals als "Web Client" in das Softwareframework eingebettet werden können. Dadurch ist es möglich, dass das Programmiergerät in ein Klinik- bzw. Praxisnetzwerk eingebunden und von allen im Netzwerk authentifizierten Rechnern genutzt werden kann.

In alternativen Ausführungsvarianten ist das MICS-Universalprogrammiergerät als USB-Stick ausgerührt und besitzt vorzugsweise die Abmessungen (B×H×T) 20×10×30mm. Hierbei ist das MICS-Universalprogrammiergerät vorzugsweise als USB-Stick inklusive integrierter MICS-Antenne ausgeführt. Vorzugsweise besitzt der USB-Stick mindestens zwei integrierte MICS-Antennen, um eine Antennen-Diversität zu ermöglichen.

Weitere vorteilhafte Ausführungsvarianten ergeben sich durch Kombination der hier erörterten bevorzugten Merkmale.

Die Erfindung soll nun anhand von Ausführungsbeispielen mit Bezug auf die Figuren näher erläutert werden. Diese zeigen in:
- Fig. 1:: Ein Blockschaltbild eines erfindungsgemäßen MICS-Band-Universalprogram- miergerätes;
- Fig. 2:: ein Blockschaltbild eines alternativen MICS-Universalprogrammiergerätes;
- Fig. 3:: die Softwarearchitektur eines MICS-Universalprogrammiergerätes;
- Fig. 4:: eine mögliche Einbindung des MICS-Universalprogrammiergerätes in ein be- stehendes Netzwerk;
- Fig. 5:: die Ausführung eines Universalprogrammiergerätes als Notfallprogrammierge- rät für den Einsatz in einem Notarztwagen oder Ambulanzflugzeug;
- Fig. 6:: die konstruktive Realisierung eines solchen modularen MICS-Universal- Programmiergerätes; und
- Fig. 7:: eine alternative Realisierung des universellen MICS-Programmiergerätes in Form eines MICS-USB-Sticks.

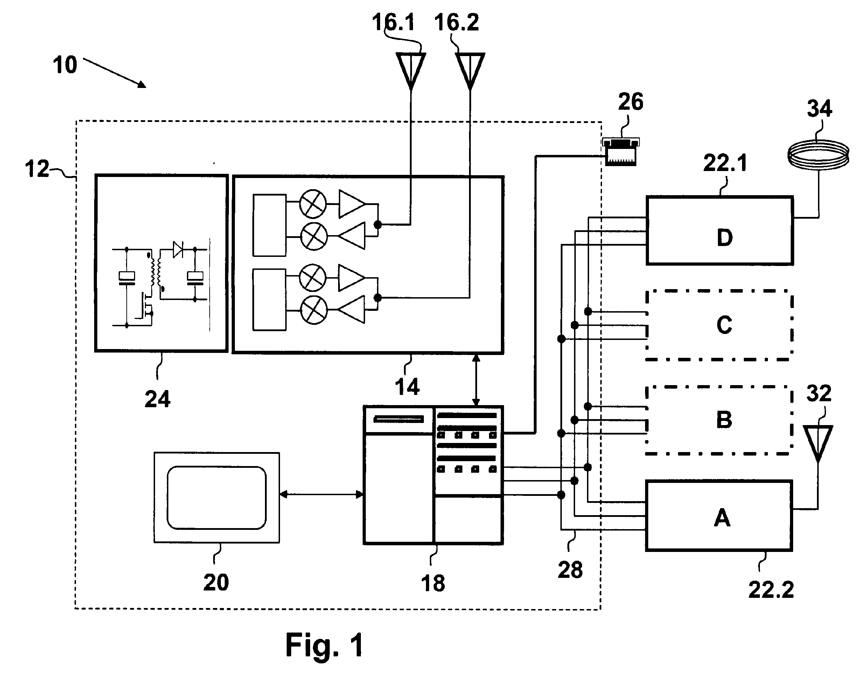

Figur 1 zeigt ein Blockschaltbild eines erfindungsgemäßen MICS-Band-Universalprogrammiergerätes 10. Das Universalprogrammiergerät weist eine MICS-Basisstation 12 auf, die einen dualen MICS-Transceiver 14 mit zwei Antennen 16.1 und 16.2 umfasst. So kann eine Antennendiversität realisiert werden.

Zur Steuerung ist eine programmierbare Steuereinrichtung 18 vorgesehen. Für die Bedienung der Basisstation 12 steht ein berührungsempfindliches Display (Touchscreen) 20 zur Verfügung. Für die Stromversorgung der Basisstation 12 und etwaiger Hardware-Zusatzmodule 22.1 oder 22.2 ist ein gemeinsames Netzteil 24 in der Basisstation 12 integriert. Die Basisstation 12 besitzt eine Ethernet-Schnittstelle 26 und kann über eine Ethernet-Verbindung in ein vorhandenes Klinik- oder Praxisnetzwerk eingebunden werden und wird vorzugsweise von einem oder mehreren Terminals dieses Netzwerkes gesteuert.

Über ein definiertes Bussystem 28 können herstellerspezifische Hardware-Zusatzmodule 22 in die Basisstation integriert werden. Diese Hardware-Zusatzmodule 22 können weitere herstellerspezifische Antennen, z.B. zur Aktivierung der MICS-Telemetrie, beinhalten bzw. die Antennen können an diese Module angeschlossen werden. Im Beispiel ist das Modul des Herstellers "A" mit einer 2,1 GHz Antenne 32 und das Modul des Herstellers "D" mit einer Spule 34 zur induktiven Kommunikation mit 175 kHz verbunden.

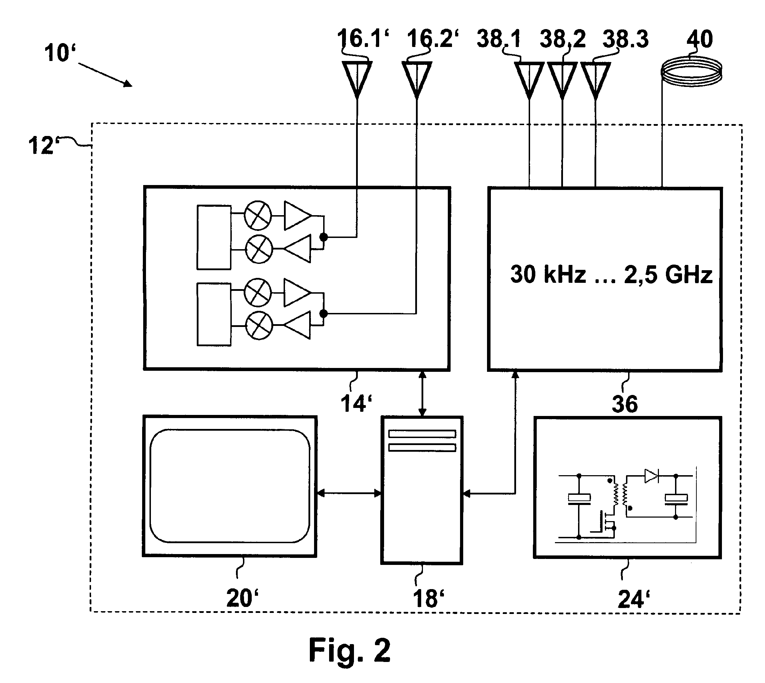

Figur 2 zeigt ein Blockschaltbild eines alternativen MICS-Universalprogrammiergerätes 10' mit einer Steuereinrichtung 18', einem daran angeschlossenen Mensch-Maschine-Interface realisiert als Touch-Screen 20', einem dualen MICS-Transceiver 14', dessen MICS-Antennen 16.1' und 16.2', einem Software-programmierbaren Radio 36 und dessen Antennen 38.1, 38.2 und 38.3 nebst einer induktiven Programmierspule 40 und einer Stromversorgungseinheit 24'.

Die MICS-Antennen 16.1' und 16.2' sind dabei derart angeordnet, dass eine Antennen-Diversität realisiert werden kann. Dies ist aus Figur 2 nicht unmittelbar ersichtlich, aber ein für den Fachmann an sich bekanntes Merkmal.

Das Software-programmierbare Radio 36 dient der Aktivierung der MICS-Kommunikation in einem elektronischen Implantat. Das Software-programmierbare Radio 36 ist mit mehreren Antennen 38.1, 38.2 und 38.3 verbunden, um so für die Aktivierung der MICS-Kommunikation die genutzten Frequenzen (~30 kHz...~2 GHz) übertragen zu können. Die Auswahl der genutzten Antenne wird dabei abhängig von der jeweils eingestellten Frequenz ausgewählt. Für die niedrigen Frequenzen wird die induktive Programmierspule ausgewählt.

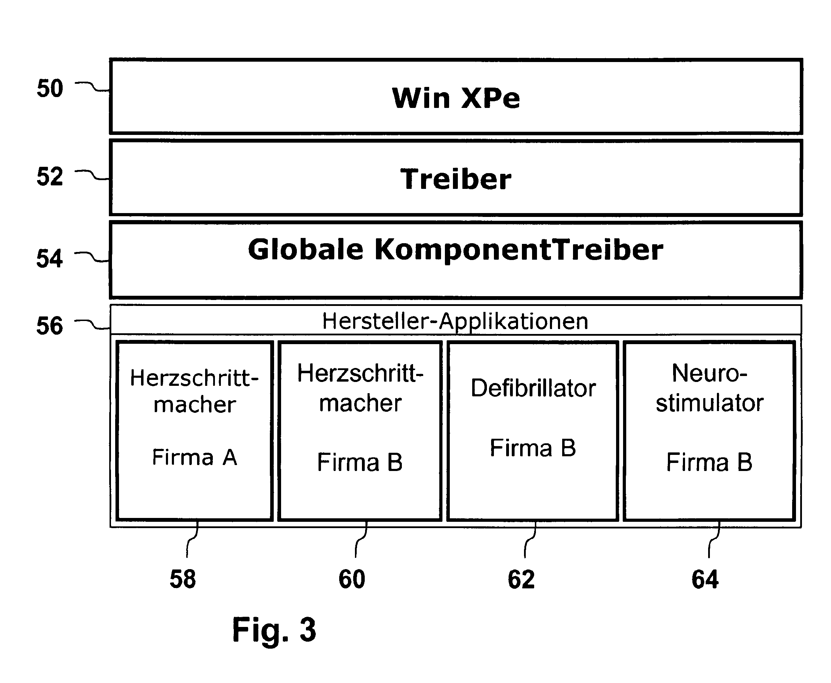

Figur 3 zeigt die Softwarearchitektur eines solchen Systems. Das Gesamtsystem basiert auf einem Windows XP-embedded Betriebssystem 50. Für die einzelnen Hardwarekomponenten sind spezifische Treiberkomponenten 52 verfügbar, die dann in sog. Globalen Komponenten Treibern 54 zusammengefasst sind und den Hersteller-Applikationen in Form von Software-Modulen 56 über definierte vorkonfigurierte Schnittstellen zugänglich gemacht sind. Als bevorzugte Softwaretechnologie wird hier COM oder .net eingesetzt.

Die Hersteller liefern dann zum universellen Programmiergerät 10 oder 10' implantatspezifische und herstellerspezifische Applikationen in Form von Software-Modulen 56. Im gezeigten Beispiel sind zwei Hersteller eingebunden. Hersteller A unterstützt mit einem entsprechenden Softwaremodul 58 einen Herzschrittmacher, Hersteller B mit entsprechenden Softwaremodulen 60, 62 und 64 einen Herzschrittmacher (Software-Modul 60), einen implantierbaren Defibrillator (Software-Modul 62) und einen Neurostimulator (Software-Modul 64).

Durch Hinzufügen derartiger Software-Module kann die Anzahl der unterstützten Implantate beliebig erweitert werden.

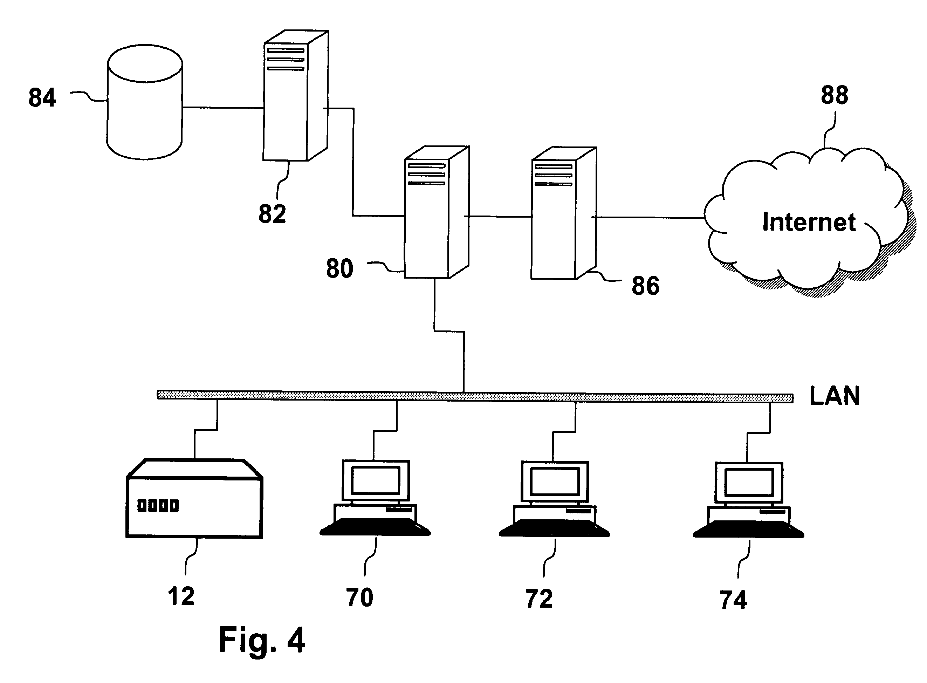

Figur 4 zeigt die mögliche Einbindung des MICS-Universalprogrammiergerätes 10 in ein bestehendes Netzwerk. Das MICS-Universalprogrammiergerät 10 ist dabei mit dem LAN der Klinik verbunden. Die Steuerung des Programmiergerätes erfolgt über im LAN verfügbare Arbeitsstationen 70, 72 und 74. Zum Beispiel kann das System derart konfiguriert werden, dass über zwei Arbeitsstationen 70 und 72, die sich im Nachsorgeraum befinden, zeitgleich der nachsorgende Arzt und eine Assistentin sich mit dem MICS-Programmiergerät 12 verbinden können und in der gleichen Sitzung der Arzt die klinischen Daten (z.B. Elektrokardiogramme, Therapieparameter) des elektronischen Implantates prüfen und verändern kann und die Assistentin die administrativen Daten (Patientendaten etc.) des elektronischen Implantates verändern kann. Die Arbeitsstation 74 wiederum hat nur einen eingeschränkten Zugriff (z.B. "Nur Lesen") auf das MICS-Programmiergerät 12 und dient der Qualitätssicherung, Arztbrieferstellung und Abrechnungserstellung (z.B. DRG-Kodierung). Diese Arbeitsstation ist in einem anderen Raum der Klinik untergebracht.

Das LAN ist in herkömmlicher Weise dann über einen Proxy-Server 80 mit einem File-Server 82 verbunden und gestattet so die automatische Patientendatenspeicherung in einer Datenbank 84 des Klinikinformationssystems. Über den Proxy-Server 80 und eine Firewall 86 ist eine Internetanbindung 88 möglich, so dass die Daten auch über Internetdienste ausgetauscht werden können.

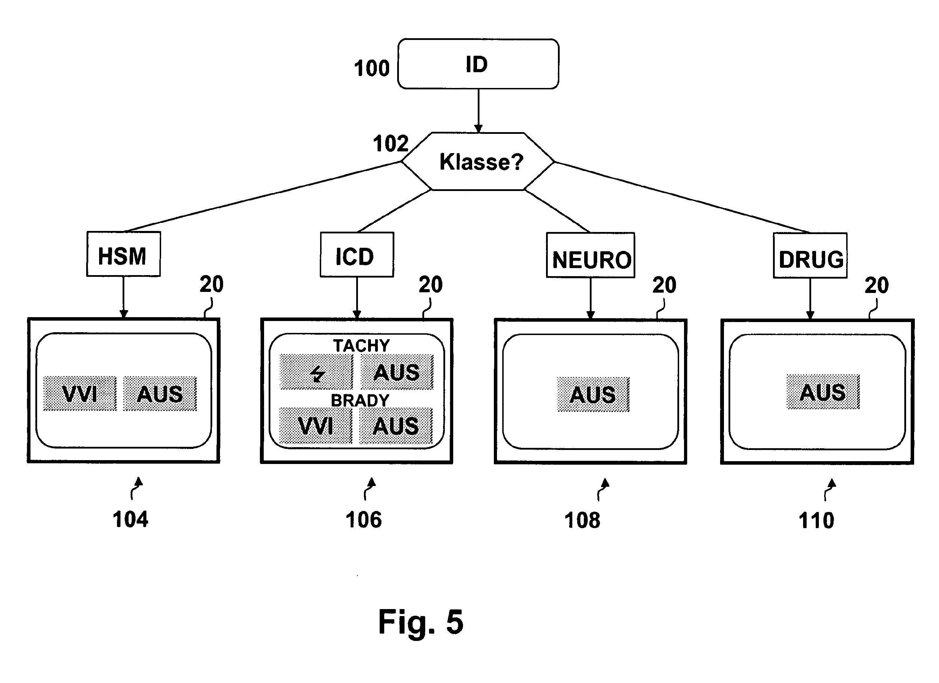

In Figur 5 ist die Ausführung eines Universalprogrammiergerätes 10 oder 10' als Notfallprogrammiergerät für den Einsatz in einem Notarztwagen oder Ambulanzflugzeug dargestellt. Dargestellt ist die innere logische Struktur eines solchen Universalprogrammiergerätes. Dieses weist einen Touchscreen 20 auf, der je nach Situation eine unterschiedliche graphische Benutzeroberfläche (GUI) bietet. Hierzu erfolgt zunächst eine automatische Identifikation 100 eines jeweiligen elektronischen Implantates. Daraufhin erfolgt eine Zuordnung 102 zu unterschiedlichen Implantatklassen. In Abhängigkeit dieser Zuordnung 102 werden je nach Implantatklasse unterschiedliche Notfallfunktionen auf dem Display 20 des Universal-Notfallprogrammiergeräts angeboten. So kann im Falle eines Herzschrittmachers ein Schutzprogramm (VVI) oder ein AUS-Mode (AUS) durch Berühren der entsprechenden Felder auf dem Touchscreen 20 programmiert werden. Die entsprechende graphische Benutzeroberfläche ist bei 104 dargestellt. Bei einem implantierbaren Defibrillator kann ein Notschock ( 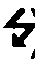 ) ausgelöst werden, die antitachykarde Therapie ausgeschaltet (z.B. bei inadäquaten Schockabgaben), ein Schrittmacherschutzprogramm gestartet oder der Schrittmacher abgeschaltet werden. Die entsprechende grafische Benutzeroberfläche zur Steuerung eines Defibrillators ist bei 106 dargestellt.

Ein Neurostimulator oder eine implantierte Medikamentenpumpe können im Notfall abgeschaltet werden (AUS). Die entsprechenden grafischen Benutzeroberflächen, die zur Steuerung eines Neurostimulators bzw. einer implantierten Medikamentenpumpe auf dem Touchscreen 20 dargestellt werden, sind bei 108 bzw. 110 dargestellt.

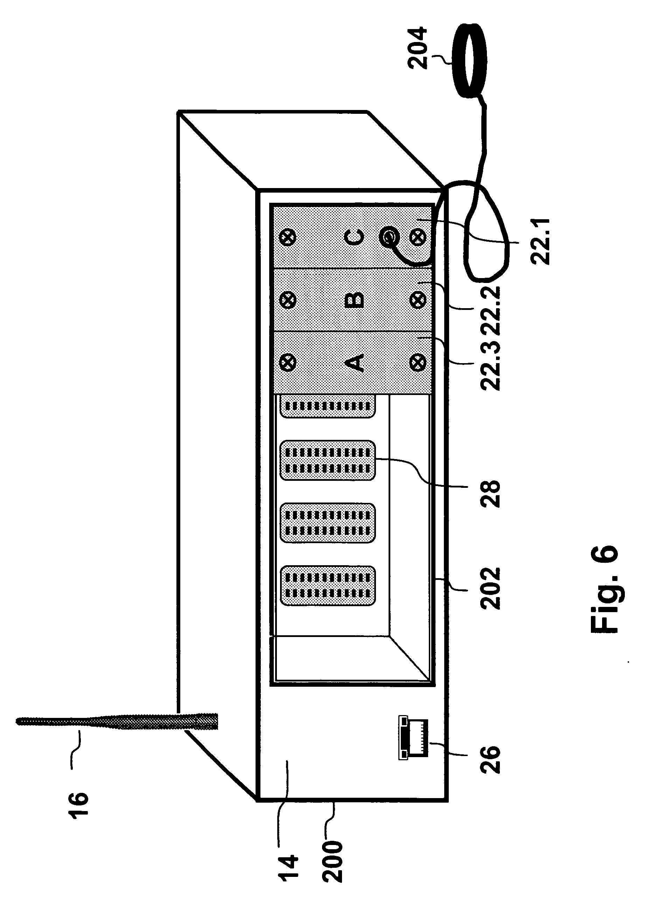

Figur 6 zeigt die konstruktive Realisierung eines solchen modularen MICS-UniversalProgrammiergerätes 10. Das Gerät wird von einem Mainframe 200 gebildet. Dieser beinhaltet einen MICS-Transceiver 14 sowie die zugehörige optimierte MICS-Band-Antenne 16. Alternativ (hier nicht dargestellt) kann ein zweiter MICS-Transceiver bereits im Mainframe enthalten sein und eine zweite Antenne angebracht sein, so dass eine Antennendiversität bereits mit dem Standard-Mainframe gegeben ist.

Zur Steuerung des Universalprogrammiergerätes 10 ist eine Ethernet-Schnittstelle 26 vorgesehen, so dass das Gerät in jedes vorhandene Klinik- oder Praxisnetzwerk eingebunden werden kann. Als Terminal dient in diesem Fall ein ohnehin in der Klinik bzw. Praxis vorhandener Personalcomputer (PC). Die zugehörige grafische Benutzeroberfläche (graphical user interface, GUI) zur Darstellung auf dem Bildschirm des jeweiligen Personalcomputers wird von den herstellerindividuellen Hardware-Zusatzmodulen 22 via Web-Applikation mitgeliefert.

Im Mainframe ist ein Schacht 202 für die Bestückung mit herstellerindividuellen Hardwaremodulen vorgesehen, die elektrische Anbindung findet dabei mit Steckerleisten 204 an der Rückseite des Schachtes 202 statt. Die Steckerleisten 204 sind mit dem Bussystem 28 (siehe Figur 1) verbunden. In der Abbildung sind 3 herstellerindividuelle Hardwaremodule 22 der Hersteller "A", "B" und "C" dargestellt. Das Modul des Hersteller "C" hat zusätzlich einen Anschluss für eine induktive Programmierspule 204 zur Aktivierung der MICS-Telemetrie im elektronischen Implantat.

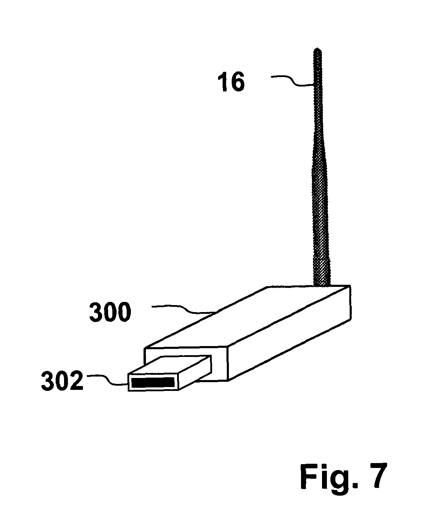

Figur 7 zeigt eine alternative Realisierung des universellen MICS-Programmiergerätes 10 in Form eines MICS-USB-Sticks 300. Dieser enthält ein universelles MICS-Band-Radio. Die MICS-Antenne 16 oder 16' ist dabei entweder in den USB-Stick integriert oder zur Optimierung der Sende- und Empfangsqualität extern an den USB-Stick angeschlossen.

Die Steuerung dieses MICS-Band-USB-Sticks erfolgt über entsprechende Softwarekomponenten. So ist es möglich, einen extrem preiswerten und universell einsetzbaren MICS-Band-Universalprogrammer zu realisieren, der über einen USB-Stecker 302 an jeden Rechner mit USB-Schnittstelle angeschlossen werden kann.

## Patentansprüche

1. Programmiergerät (10) für patientenindividuelle Medizingeräte, wie Implantate, mit:
- einem RF-Transceiver (Sender/Empfänger) (14), der zum Empfang und zur Übertragung von Daten im MICS-Frequenzband ausgebildet ist,
- einer oder mehreren Antennen (16, 16') zur MICS Kommunikation, die mit dem RF-Transceiver (14, 14') verbunden sind.
- einer Steuereinrichtung (18), die mit dem RF-Transceiver (14) verbunden ist und
- einer mit der Steuereinrichtung (18) verbundenen Mensch-Maschine-Schnittstelle (20) oder einem mit der Steuereinrichtung (18) verbundenen Anschluss für eine Mensch-Maschine-Schnittstelle (20);
**dadurch gekennzeichnet, dass**
der RF-Transceiver (14) derart ausgebildet ist, dass er für Modulations- und Protokollverfahren im MICS Band geeignet ist und unterschiedliche MICS-Protokolle unterstützen kann und
die Steuereinrichtung (18) derart vorkonfigurierte Softwareschnittstellen (54) aufweist, dass das Programmiergerät (10) mit Steuersoftwaremodulen (56) erweiterbar ist, wobei die vorkonfigurierten Softwareschnittstellen (54) eine einheitliche Schnittstelle zur Ansteuerung des RF-Transceivers (14) definieren, auf die Steuersoftwaremodule (56) zugreifen können und
die Steuereinrichtung (18) einheitliche Hardware-Schnittstellen (28) für mehrere Hardware-Zusatzmodule (22.1, 22.2) aufweist, die über die Steuereinrichtung (18) mit dem RF-Transceiver (14) verbunden sind, und jeweils eine Treibersoftware zur Ansteuerung der Antennen (16, 16') enthalten.

2. Programmiergerät (10) nach Anspruch 1, **dadurch gekennzeichnet, dass** die vorkonfigurierten Softwareschnittstellen (54) derart konfiguriert sind, dass die elektronischen Implantate verschiedener Hersteller durch Hinzufügen von implantatspezifischen Softwarekomponenten mindestens identifiziert werden können.

3. Programmiergerät (10) nach einem der Ansprüche 1 bis 2, **dadurch gekennzeichnet, dass** die Hardware-Zusatzmodule (22) bereits eine jeweilige implantatsspezifische Software einschließlich der Komponenten für ein grafisches Benutzerinterface (GUI) zur Wiedergabe über die Mensch-Maschine-Schnittstelle (20) beinhalten.

4. Programmiergerät (10) nach einem der Ansprüche 1 bis 3, **dadurch gekennzeichnet, dass** die Mensch-Maschine-Schnittstelle (20) eine oder mehrere Tasten aufweist, die zur Auslösung implantatspezifischer Notfallfunktionen mit der Steuereinrichtung verbunden sind.

5. Programmiergerät (10) nach einem der Ansprüche 1 bis 4, **dadurch gekennzeichnet, dass** das Programmiergerät (10) zusätzliche Frequenzgeneratoren (36) für eine Datenkommunikation in anderen Frequenzbereichen als dem MICS-Frequenzbereich aufweist.

6. Programmiergerät (10) nach einem der Ansprüche 1 bis 5, **dadurch gekennzeichnet, dass** das Programmiergerät (10) diesem fest zugeordnete Softwareframeworks und definierte Schnittstellen zu Steuersoftwaremodulen aufweist.

7. Programmiergerät (10) nach einem der Ansprüche 1 bis 6, **dadurch gekennzeichnet, dass** das Programmiergerät (10) eine oder mehrere Schnittstellen zu einem oder mehreren Terminals zur Steuerung der Funktionen durch einen Anwender besitzt.

8. Programmiergerät (10) nach den Ansprüchen 6 und 7, **dadurch gekennzeichnet, dass** das Programmiergerät (10) derart ausgebildet ist, dass die Terminals als Web Client in das Softwareframework eingebettet werden können.

## Claims

1. A programmer (10) for patient medical devices such as implants, comprising:
- an RF transceiver (transmitter/receiver) (14) which is designed to receive and transmit data in the MICS frequency band,
- one or more antennas (16, 16') for MICS communication, which are connected to the RF transceiver (14, 14').
- a control device (18) which is connected to the RF transceiver (14), and
- a human-machine interface (20), which is connected to the control device (18), or a connection for a human-machine interface (20), which is connected to the control device (18);
**characterized in that**
the RF transceiver (14) is designed such that it is suitable for modulation and protocol processes in the MICS band and can support different MICS protocols, and
the control device (18) comprises software interfaces (54) which have been preconfigured in such a way that the programmer (10) can be expanded with control software modules (56), wherein the preconfigured software interfaces (54) define a uniform interface for controlling the RF transceiver (14), which can be accessed by control software modules (56), and
the control device (18) comprises uniform hardware interfaces (28) for a plurality of additional hardware modules (22.1, 22.2) which are connected to the RF transceiver (14) by way of the control device (18), and each of which contains driver software for controlling the antennas (16, 16').

2. The programmer (10) according to claim 1, **characterized in that** the preconfigured software interfaces (54) are configured in such a way that the electronic implants from various manufacturers can be identified, at the least, by adding implant-specific software components.

3. The programmer (10) according to one of the claims 1 to 2, **characterized in that** the additional hardware modules (22) already contain particular implant-specific software, including components for a graphical user interface (GUI) for reproduction by way of the human-machine interface (20).

4. The programmer (10) according to one of the claims 1 to 3, **characterized in that** the human-machine interface (20) comprises one or more keys which are connected to the control device for triggering implant-specific emergency functions.

5. The programmer (10) according to one of the claims 1 to 4, **characterized in that** the programmer (10) comprises additional frequency generators (36) for data communication in frequency ranges other than the MICS frequency range.

6. The programmer (10) according to one of the claims 1 to 5, **characterized in that** the programmer (10) comprises software frameworks fixedly assigned thereto, and defined interfaces to control software modules.

7. The programmer (10) according to one of the claims 1 to 6, **characterized in that** the programmer (10) comprises one or more interfaces to one or more terminals to permit control of the functions by a user.

8. The programmer (10) according to one of the claims 6 and 7, **characterized in that** the programmer (10) is designed in such a way that the terminals can be embedded in the software framework as a web client.

## Revendications

1. Dispositif de programmation (10) pour des appareils médicaux individuels, tels que des implants, avec :
- un émetteur-récepteur RF (14) conçu pour la réception et la transmission de données dans la bande de fréquences MICS,
- une ou plusieurs antennes (16, 16') reliées à l'émetteur-récepteur RF (14, 14'), pour la communication MICS,
- un dispositif de commande (18) relié à l'émetteur-récepteur RF (14), et
- une interface homme-machine (20) reliée au dispositif de commande (18), ou un raccord relié au dispositif de commande (18) pour une interface homme-machine (20) ;
**caractérisé en ce que**
- l'émetteur-récepteur RF (14) est conçu de manière à s'adapter à tous les procédés pour la modulation et les protocoles dans la bande MICS, et à pouvoir soutenir différents protocoles MICS, et
- le dispositif de commande (18) comporte des interfaces de logiciels (54) préconfigurées de telle manière, que le dispositif de programmation (10) peut être étendu avec des modules de logiciel de commande (56), où les interfaces de logiciels préconfigurées (54) définissent une interface unique pour l'actionnement de l'émetteur-récepteur RF (14), à laquelle les modules de logiciel de commande (56) peuvent accéder, et
- le dispositif de commande (18) comporte des interfaces de matériel homogènes (28) pour plusieurs modules supplémentaires de matériel (22.1, 22.2), qui sont reliées à l'émetteur-récepteur RF (14) par le dispositif de commande (18) et contiennent respectivement un logiciel pilote pour l'actionnement des antennes (16, 16').

2. Dispositif de programmation (10) selon la revendication 1, **caractérisé en ce que** les interfaces de logiciel préconfigurées (54) sont configurées de telle manière, que les implants électroniques de différents fabricants peuvent au moins être identifiés, par l'ajout de composants logiciels spécifiques aux implants.

3. Dispositif de programmation (10) selon l'une des revendications 1 à 2, **caractérisé en ce que** les modules supplémentaires de logiciel (22) contiennent déjà un logiciel spécifique à l'implant, y compris les composants pour une interface utilisateur graphique (GUI), pour la restitution par l'interface homme-machine (20).

4. Dispositif de programmation (10) selon l'une des revendications 1 à 3, **caractérisé en ce que** l'interface homme-machine (20) comporte une ou plusieurs touches reliées au dispositif de commande pour l'activation de fonctions d'urgence spécifiques à l'implant.

5. Dispositif de programmation (10) selon l'une des revendications 1 à 4, **caractérisé en ce que** le dispositif de programmation (10) comporte des générateurs de fréquence (36) supplémentaires, pour une transmission de données dans d'autres plages de fréquences que la plage de fréquences MICS.

6. Dispositif de programmation (10) selon l'une des revendications 1 à 5, **caractérisé en ce que** le dispositif de programmation (10) comporte des infrastructures de logiciel qui lui sont fixement attribués, ainsi que des interfaces définis pour des modules de logiciel de commande.

7. Dispositif de programmation (10) selon l'une des revendications 1 à 6, **caractérisé en ce que** le dispositif de programmation (10) possède une ou plusieurs interfaces pour un ou plusieurs terminaux destinés à la commande des fonctions par un utilisateur.

8. Dispositif de programmation (10) selon les revendications 6 et 7, **caractérisé en ce que** le dispositif de programmation (10) est conçu de telle manière, que les terminaux peuvent être intégrés en tant que Client Web dans l'infrastructure de logiciel.
